(19) Europäisches Patentamt
European Patent Office
Office européen des brevets

(11) Veröffentlichungsnummer: **0 471 232 A1**

(12) **EUROPÄISCHE PATENTANMELDUNG**

(21) Anmeldenummer: **91112669.6**

(51) Int. Cl.5: **C07D 271/04**, A61K 31/41

(22) Anmeldetag: **27.07.91**

(30) Priorität: **13.08.90 DE 4025604**

(43) Veröffentlichungstag der Anmeldung: **19.02.92 Patentblatt 92/08**

(84) Benannte Vertragsstaaten: **AT BE CH DE DK ES FR GB GR IT LI NL SE**

(71) Anmelder: **CASSELLA Aktiengesellschaft**
**Hanauer Landstrasse 526**
**W-6000 Frankfurt am Main 60(DE)**

(72) Erfinder: **Schönafinger, Karl, Dr.**
**Holunderweg 8**
**W-8755 Alzenau(DE)**
Erfinder: **Beyerle, Rudi, Dr.**
**An der Pfaffenmauer 44**
**W-6000 Frankfurt 60(DE)**
Erfinder: **Bohn, Helmut, Dr.**
**Kranzbergring 11**
**W-6369 Schöneck(DE)**
Erfinder: **Just, Melitta, Dr.**
**Theodor-Heuss-Strasse 80**
**W-6070 Langen 2(DE)**

(74) Vertreter: **Urbach, Hans-Georg, Dr. et al**
**CASSELLA AKTIENGESELLSCHAFT**
**Patentabteilung Hanauer Landstrasse 526**
**W-6000 Frankfurt am Main 60(DE)**

(54) **3-Dicyclohexylamino-sydnonimine, Verfahren zu ihrer Herstellung und ihre Verwendung.**

(57) Die vorliegende Erfindung betrifft pharmakologisch wirksame substituierte 3-Dicyclohexylamino-sydnonimine der allgemeinen Formel I

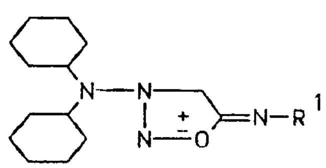

(I)

und ihre pharmakologisch annehmbaren Säureadditionssalze, worin $R^1$ Wasserstoff oder den Rest $-COR^2$ und $R^2$ $(C_1$ bis $C_4)$Alkyl, $(C_1$ bis $C_4)$Alkoxy-$(C_1$ bis $C_4)$alkyl, $(C_1$ bis $C_4)$Alkoxy, $(C_1$ bis $C_4)$Alkoxy-$(C_1$ bis $C_4)$alkoxy, $(C_5$ bis $C_7)$Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest, einen Nicotinoylrest oder einen Allylmercaptoacetylrest bedeuten, sowie ein Verfahren zur Herstellung der erfindungsgemäßen Verbindungen und ihre Verwendung.

EP 0 471 232 A1

Die Erfindung betrifft pharmakologisch wirksame substituierte 3-Dicyclohexylamino-sydnonimine der allgemeinen Formel I

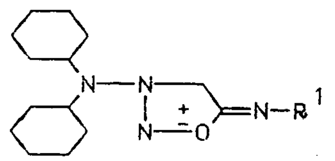

( I )

und ihre pharmakologisch annehmbaren Säureadditionssalze, worin $R^1$ Wasserstoff oder den Rest $-COR^2$ und $R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_1$ bis $C_4$)Alkoxy-($C_1$ bis $C_4$)alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest, einen Nicotinoylrest oder einen Allylmercaptoacetylrest bedeuten.

Die Erfindung betrifft ferner ein Verfahren zur Herstellung der erfindungsgemäßen Verbindungen und ihre Verwendung.

Mit den erfindungsgemäßen Verbindungen strukturell verwandte Verbindungen sind bereits in der DE-A-16 70 127 beschrieben.

Alkylreste, Alkoxyalkylreste, Alkoxyreste und Alkoxyalkoxyreste können geradkettig oder verzweigt sein. Dies gilt auch, wenn sie als Substituenten für Phenyl auftreten.

($C_1$ bis $C_4$)Alkylreste können sein: Methyl, Ethyl, n-Propyl, i-Propyl, n-Butyl, i-Butyl, tert.-Butyl, sec.-Butyl.

($C_1$ bis $C_4$)Alkoxyreste können z.B. sein: Methoxy, Ethoxy, n-Propoxy, i-Propoxy, n-Butoxy, i-Butoxy, sec.-Butoxy, tert.-Butoxy.

($C_1$ bis $C_4$)Alkoxy-($C_1$ bis $C_4$)alkylreste können z.B. sein: Methoxy-methyl, 2-Methoxy-ethyl, 3-Methoxy-propyl, 4-Methoxy-butyl, 2-Ethoxy-ethyl, 3-Propoxy-propyl, 4-Propoxy-butyl, 2-Butoxy-ethyl, 3-Butoxy-propyl, 4-Butoxy-butyl, 3-i-Propoxy-propyl, 4-i-Propoxy-butyl.

($C_1$ bis $C_4$)Alkoxy-($C_1$ bis $C_4$)alkoxyreste können z.B. sein: Methoxy-methoxy, 2-Methoxy-ethoxy, 3-Methoxy-propoxy, 4-Methoxy-butoxy, 2-Ethoxy-ethoxy, 3-Propoxy-propoxy, 4-Propoxy-butoxy, 2-Butoxy-ethoxy, 3-Butoxy-propoxy, 4-Butoxy-butoxy, 3-i-Propoxy-propoxy, 4-i-Propoxy-butoxy, 2-i-Propoxy-ethoxy.

Von den ($C_1$ bis $C_5$)Cycloalkylresten sind Cyclopentyl und Cyclohexyl bevorzugt.

Als Halogenatome für den substituierten Phenylrest kommen Fluor, Chlor, Brom und/oder Iod in Betracht, von denen Brom und Chlor bevorzugt sind.

Der für $R^2$ stehende substituierte Phenylrest ist vorzugsweise monosubstituiert, insbesondere in 2-oder 4 Stellung und/oder durch Methoxy oder Chlor. Bevorzugte Reste für $R^2$ sind Ethoxy, p-Methoxyphenyl, p-Chlorphenyl, tert-Butyl, 2-Isopropoxyethoxy und Allylmercaptoacetyl.

Eine Verbindung der allgemeinen Formel I kann dadurch hergestellt werden, daß die Verbindung der Formel II

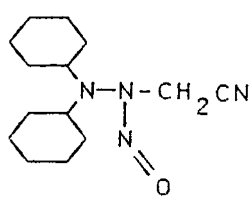

( II )

zu der Verbindung der allgemeinen Formel Ia

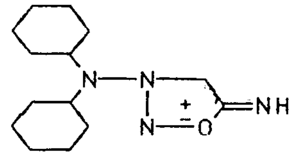

( Ia )

cyclisiert wird und daß diese oder ein Säureadditionssalz davon für den Fall, daß eine Verbindung der

Formel I mit $R^1$ = $-COR^2$ hergestellt werden soll, mit einem Acylierungsmittel, das den Rest $-COR^2$ einführt, acyliert wird und die so erhaltene Verbindung gegebenenfalls in ein pharmakologisch annehmbares Säureadditionssalz überführt wird.

Die Cyclisierung der Verbindung II zu der Verbindung Ia wird in einem geeigneten organischen oder anorganischen Lösungs-, Dispergier- oder Verdünnungsmittel unter Zusatz eines Cyclisierungsmittels, normalerweise bei Temperaturen von - 10 bis 40 °C, insbesondere 0 bis 40 °C, vorzugsweise bei 0 bis 20 °C, durchgeführt.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butyl-ether, Tetrahydrofuran, 1,4-Dioxan, 1,2-Dimethoxyethan, Bis-$\beta$-methoxyethylether; Oligoethylen-glykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butylester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig- und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugs weise Essigsäureethylester-Methanol.

Die Verbindung der Formel Ia stellt die erfindungsgemäße Verbindung der allgemeinen Formel I für den Fall dar, daß $R^1$ = Wasserstoff ist.

Die Acylierung der Verbindung der Formel Ia, die auch in Form eines Säureadditionssalzes vorliegen kann, zur Einführung des Restes $R^1$ = $COR^2$ kann in an sich bekannter Weise mit einem geeigneten Acylierungsmittel der Formel III durchgeführt werden

$$X-\overset{\overset{O}{\|}}{C}-R^2 \qquad (III)$$

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^2$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger oder flüssiger disperser Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III theoretisch 1 : 1. Das Acylierungsmittel kann jedoch auch im Über- oder Unterschuß eingesetzt werden. Zweckmäßigerweise wird das Acylierungsmittel der Formel III im Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium-oder Lithium-hydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen: Säurehalogenide bzw. Halogenameisensäureester, von denen Säurechloride und Chlorameisensäureester bevorzugt sind; für -OH: Carbonsäuren; für -O-Alkyl und -O-Aryl: Ester, von denen die Tolyl-, 2,4-Dinitro- oder 4-Nitrophenylester bevorzugt sind; für -O-CO-$R^2$: Anhydride; für -O-CO-OAlkyl: gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1264 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere denn sie mindestens einen sekundären oder tertiären Alkylrest enthalten, wie z.B. Diisopropyl-, Dicyclohexyl- oder N-Methyl-N'-tert.butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimido-carbonat, Diphthalimido-carbonat, 1,1'-(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N'-Carbonyldiazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die Verbindungen der allgemeinen Formel I können mit anorganischen oder organischen Säuren Säureadditionssalze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindung der Formel Ia fallen normalerweise die Säureadditionssalze an. Aus den Säureadditionssalzen kann die freie Verbindung der allgemeinen Formel Ia gewünschtenfalls in bekannter Weise, d.h. durch Auflösen oder Suspendieren in Wasser und Alkalischstellen, z.B. mit Natronlauge, und anschließendes Isolieren, gewonnen werden.

Die Ausgangsverbindung der Formel II kann in einfacher und an sich bekannter Weise nach folgendem Reaktionsschema hergestellt werden:

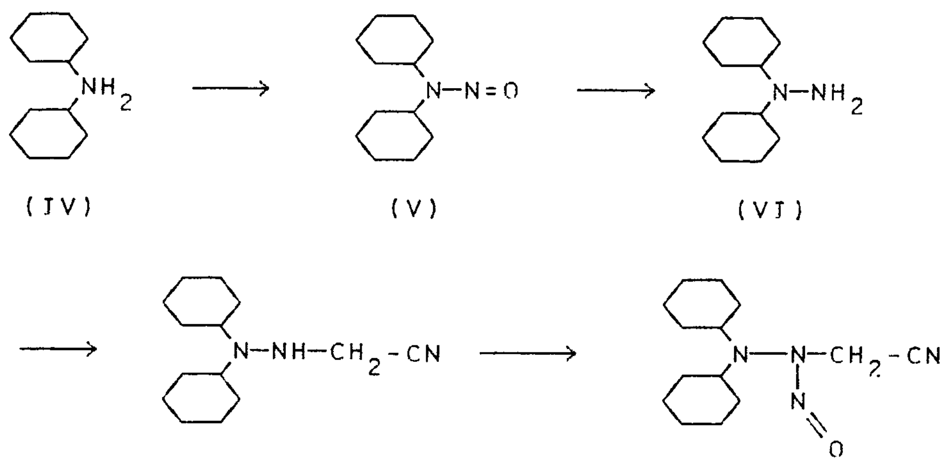

(IV) (V) (VI)

(VII) (II)

Danach wird Dicyclohexylamin der Formel IV mit salpetriger Säure nitrosiert und das erhaltene N-Nitroso-dicyclohexylamin der Formel V zum N,N-Dicyclohexylhydrazin der Formel VI reduziert. Dieses wird zur Verbindung der Formel VII cyanmethyliert und schließlich zur Verbindung der Formel II nitrosiert.

Die Nitrosierungen werden in bekannter Weise, vorzugsweise in Wasser, z.B. bei Temperaturen von 0 bis 10 °C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Vorstufen mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Reduktion der Verbindung der Formel V zur Verbindung der Formel VI wird in an sich bekannter Weise mit Lithiumalanat, mit Zn-Staub in Eisessig oder mit Natrium in Alkohol durchgeführt. Aber auch andere literaturbekannte Verfahren lassen sich anwenden.

Die Cyanmethylierung zur Verbindung der Formel VII gelingt in ebenfalls bekannter Weise durch Umsetzung der Verbindung der Formel VI mit Formaldehyd und Blausäure bzw. Natriumcyanid in einem geeigneten Lösungsmittel, z.B. Wasser.

Die Verbindung der Formel IV ist käuflich.

Die Verbindungen der allgemeinen Formel I und ihre pharmakologisch annehmbaren Säureadditionssalze besitzen wertvolle pharmakologische z.B. hämodynamische, trombozytenfunktionshemmende und antithrombotische, insbesondere coronar-antithrombotische Eigenschaften. Besonders ausgeprägt ist ihre Wirkung auf das Herz-Kreislauf-System. Verglichen mit bekannten, in 3-Stellung strukturähnlichen substituierten Sydnoniminverbindungen, besitzen sie überraschenderweise eine erstaunlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eig nen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihyper tensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). Die Wirkungsdauer der Prüfsubstanz ergibt sich aus der Zeit, die nach der Zugabe der Prüfsubstanz benötigt wird, bis der Ausgangswert wieder erreicht ist. In der folgenden Tabelle sind die so erhaltenen Werte angegeben.

| | $IC_{50}$ | Wirkungsdauer in Minuten |
|---|---|---|
| 3-Dicyclohexylamino-sydnonimin-hydrochlorid | $4.10^{-7}$ | 270 |
| N-p-Anisoyl-3-dicyclohexylamino-sydnonimin | $3.10^{-6}$ | 300 |
| N-Pivaloyl-3-dicyclohexylamino-sydnonimin | $5.10^{-6}$ | 310 |
| 1.Vergleichssubstanz: Molsidomin | $3.10^{-4}$ | 120 |
| 2.Vergleichssubstanz: SIN-1 | $1.10^{-6}$ | 90 |

Molsidomin = N-Ethoxycarbonyl-3-morpholino-sydnonimin

SIN-1 = 3-Morpholino-sydnonimin-hydrochlorid

Beispiele

1. 3-Dicyclohexylamino-sydnonimin-hydrochlorid

a) 2. N-Nitroso-dicyclohexylamin

Zur Mischung aus 36,2 g Dicyclohexylamin, 18 ml konz. HCl und 300 ml Wasser wird langsam die Lösung von 20,7 g Natriumnitrit in 60 ml Wasser bei RT getropft. Darauf wird der Ansatz 7 h lang auf 95°C erhitzt. Die Nitroverbindung wird mit Ether ausgeschüttelt. Nach dem Trocknen über $Na_2SO_4$ und Abdampfen des Ethers verbleibt ein öliger Rückstand, der alsbald kristallisiert.
Ausbeute: 23 g Fp.:102 bis 103°C

b) N,N-Dicyclohexyl-hydrazin-hydrochlorid

Zur Mischung aus 126 g N-Nitroso-dicyclohexylamin, 1,2 l Dibutylether und 300 ml Tetrahydrofuran wird unter Stickstoff als Inertgas allmählich bei 90°C insgesamt 25 g Lithiumalanat zugefügt und anschließend bis zum Ende der Reaktion bei 90°C gerührt Nach Abkühlen auf 5°C wird vorsichtig mit 50 ml Wasser hydrolysiert, die festen Bestandteile abgesaugt und das Filtrat im Vakuum eingeengt. Es verbleibt ein öl, das durch Aufläsen in Ether und Einleiten von HCl-Gas ins Hydrochlorid überführt wird.
Ausbeute: 133 g Fp.:124°C (Zers.)

c) N,N-Dicyclohexylamino-aminoacetonitril

Die Suspension von 2,5 g N,N-Dicyclohexylhydrazin-hydrochlorid in 75 ml $H_2O$ wird mit 0,75 g Natriumcyanid und mit 1,2 g einer 39 %igen Formalinlösung versetzt. Diese Mischung wird bei pH = 6 bis 7 bei RT 4 h gerührt. Durch Ausschütteln mit Essigester wird das Produkt abgetrennt und nach dem Trocknen der organischen Phase als Hydrochlorid gefällt.
Ausbeute: 1,5 g Fp.:173°C (Zers.)

d) 3-Dicyclohexylamino-sydnonimin-hydrochlorid

Die im Beispiel 1c hergestellte Verbindung (1,5 g) wird unter Stickstoff in 50 ml Wasser gelöst. Nach Zugabe von 50 ml Essigester wird mit 0,6 g $NaNO_2$ versetzt und ein pH von 1 bis 2 eingestellt. Nach beendeter Reaktion wird die Essigesterphase abgetrennt, über $Na_2SO_4$ getrocknet und das Produkt durch Zugabe von überschüssiger isopropanolischer Salzsäure ausgefällt.
Ausbeute: 1,0 g Fp.:154°C (Zers.)

2. N-Pivaloyl-3-dicyclohexylamino-sydnonimin

Die Mischung von 3 g 3-Dicyclohexylamino-sydnonimin-hydrochlorid (Beispiel 1d), 50 ml Wasser und 2,1 g Natriumbicarbonat wird bei 0 bis 5°C mit der Lösung von 1,5 g Pivaloylchlorid in 40 ml Methylenchlorid vereinigt- und bei aufsteigender Temperatur 6 h lang gerührt. Die organische Phase wird abgetrennt, über $MgSO_4$ getrocknet und eingeengt. Der verbleibende Rückstand wird aus Petrolether umkristallisiert.
Ausbeute: 1,96 g Fp.:110°C
In analoger Weise wurde hergestellt:

3. N-Isopropoxyethoxycarbonyl-3-dicyclohexylamino-sydnonimin

FP.: 107°C durch Reaktion mit Chlorameisensäureisopropoxyethylester

4. N-p-Chlorbenzoyl-3-dicyclohexylamino-sydnonimin

FP.: 112 bis 114°C durch Reaktion mit p-Chlorbenzoylchlorid

5. N-Ethoxycarbonyl-3-dicyclohexylamino-sydnonimin

Fp.: 92°C durch Reaktion mit Chlorameisensäureethylester

6. N-p-Anisoyl-3-dicyclohexylamino-sydnonimin

Fp.: 160°C durch Reaktion mit p-Anissäurechlorid

7. N-Allylmercaptoacetyl-3-dicyclohexylamino-sydnonimin

Fp.: Öl durch Reaktion mit Allylmercaptoacetylchlorid

8. N-Nicotinoyl-3-dicyclohexylamino-sydnonimin

Fp.: 170 bis 172°C durch Reaktion mit Nicotinsäurechlorid

## Patentansprüche

**Patentansprüche für folgende Vertragsstaaten : AT, BE, CH/LI, DE, DK, FR, GB, IT, NL, SE**

**1.** Substituierte 3-Dicyclohexylamino-sydnonimine der allgemeinen Formel I

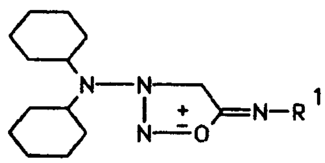

(I)

und ihre pharmakologisch annehmbaren Säureadditionssalze, worin $R^1$ Wasserstoff oder den Rest $-COR^2$ und $R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_1$ bis $C_4$)-Alkoxy-($C_1$ bis $C_4$)alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest, einen Nicotinoylrest oder einen Allylmercaptoacetylrest bedeuten.

**2.** Substituierte 3-Dicyclohexylamino-sydnonimine gemäß Anspruch 1, dadurch gekennzeichnet, daß $R^2$ Ethoxy, p-Methoxyphenyl, p-Chlorphenyl, tert-Butyl, 2-Isopropoxyethoxy oder Allylmercaptoacetyl bedeutet.

**3.** Verfahren zur Herstellung eines substituierten 3-Dicyclohexylamino-sydnonimins der allgemeinen Formel I gemäß Anspruch 1 und/oder 2, dadurch gekennzeichnet, daß die Verbindung der Formel II

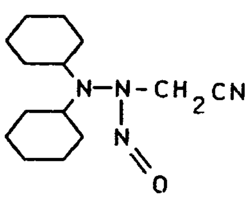

(II)

zu der Verbindung der Formel Ia

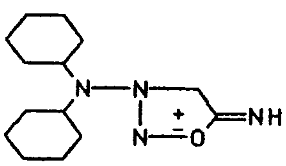

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung

der allgemeinen Formel I mit $R^1$ = $-COR^2$ hergestellt wird, die Verbindung der Formel Ia oder ein Säureadditionssalz davon mit einem Acylierungsmittel acyliert wird, das den Rest $-COR^2$ einführt und die erhaltene Verbindung gegebenenfalls in ein Säureadditionssalz überführt wird.

4. Verfahren nach Anspruch 3, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

5. Verwendung substituierter 3-Dicyclohexylamino-sydnonimine der allgemeinen Formel I gemäß Anspruch 1 und/oder 2 oder pharmakologisch annehmbare Säureadditionssalze zur Herstellung von Arzneimitteln zur Bekämpfung und Vorbeugung cardiovaskulärer Erkrankungen.

6. Pharmazeutisches Präparat, dadurch gekennzeichnet, daß es ein substituiertes 3-Dicyclohexylamino-sydnonimin der allgemeinen Formel I gemäß Anspruch 1 und/oder 2 oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.

**Patentansprüche für folgende Vertragsstaaten : ES, GR**

1. Verfahren zur Herstellung eines substituierten 3-Dicyclohexylamino-sydnonimins der allgemeinen Formel I

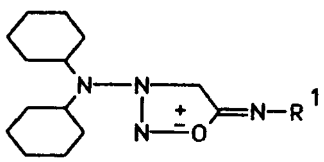

(I)

und ihrer pharmakologisch annehmbaren Säureadditionssalze, worin $R^1$ Wasserstoff oder den Rest $-COR^2$ und $R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_1$ bis $C_4$)-Alkoxy-($C_1$ bis $C_4$)alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest, einen Nicotinoylrest oder einen Allylmercaptoacetylrest bedeuten, dadurch gekennzeichnet, daß die Verbindung der Formel II

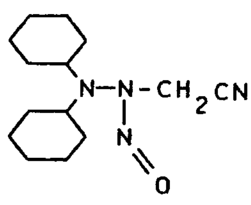

(II)

zu der Verbindung der Formel Ia

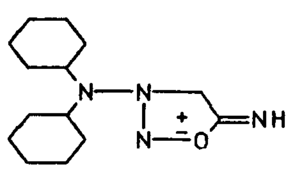

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der allgemeinen Formel I mit $R^1$ = $-COR^2$ hergestellt wird, die Verbindung der Formel Ia oder ein

Säureadditionssalz davon mit einem Acylierungsmittel acyliert wird, das den Rest $-COR^2$ einführt und die erhaltene Verbindung gegebenenfalls in ein Säureadditionssalz überführt wird.

2. Verfahren gemäß Anspruch 1, dadurch gekennzeichnet, daß in der allgemeinen Formel I $R^2$ Ethoxy, p-Methoxyphenyl, p-Chlorphenyl, tert-Butyl, 2-Isopropoxyethoxy oder Allylmercaptoacetyl bedeutet.

3. Verfahren gemäß Anspruch 1 und/oder 2, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

4. Verwendung substituierter 3-Dicyclohexylamino-sydnonimine der allgemeinen Formel I gemäß Anspruch 1 und/oder 2 oder pharmakologisch annehmbare Säureadditionssalze zur Herstellung von Arzneimitteln zur Bekämpfung und Vorbeugung cardiovaskulärer Erkrankungen.

5. Verfahren zur Herstellung einer pharmazeutischen Zubereitung enthaltend ein substituiertes 3-Dicyclohexylamino-sydnonimin der allgemeinen Formel I gemäß einem oder mehreren der Ansprüche 1 bis 3, oder ein pharmakologisch annehmbares Säureadditionssalz davon, dadurch gekennzeichnet, daß man dieses zusammen mit einem physiologisch unbedenklichen Träger, gegebenenfalls weiteren Hilfs- und Zusatzstoffen sowie gegebenenfalls einem oder mehreren anderen pharmakologischen Wirkstoffen in eine geeignete Darreichungsform bringt.

Europäisches Patentamt

# EUROPÄISCHER RECHERCHENBERICHT

Nummer der Anmeldung

EP 91112669.6

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
| D,X | DE - A1 - 1 670 127 (BOEHRINGER) * Ansprüche 1,8,9 * -- | 1,5,6 | C 07 D 271/04<br>A 61 K 31/41 |
| X | EP - A1 - 0 206 219 (CASSELLA) * Anspruch 1 * -- | 1 | |
| A | DE - A1 - 3 107 933 (CASSELLA) * Ansprüche 1,13,14,16 * -- | 1,5,6 | |
| A | DE - A1 - 3 819 914 (CASSELLA) * Ansprüche 1,10 * -- | 1,5,6 | |
| A | DE - A1 - 3 837 327 (CASSELLA) * Ansprüche 1,10 * -- | 1,5,6 | |
| A | CHEMICAL ABSTRACTS, Band 107, Nr. 3, 20. Juli 1987, Columbus, Ohio, USA W. R. KUKOVETZ et al. "Mechanism of the vasodilator and platelet aggregation--inhibiting effects of molsidomine and SIN-1" Seite 46, Spalte 2, Zusammenfassung-Nr. 17 523y & Pathol. Biol. 1987, 35(2 bis), 260-5 ---- | 1,5,6 | RECHERCHIERTE SACHGEBIETE (Int. Cl.5)<br>C 07 D 271/00 |

Der vorliegende Recherchenbericht wurde für alle Patentansprüche erstellt.

| Recherchenort | Abschlußdatum der Recherche | Prüfer |
|---|---|---|
| WIEN | 04-11-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument